# EUROPÄISCHE PATENTSCHRIFT

(11) **EP 2 432 558 B1**
(45) Veröffentlichungstag und Bekanntmachung des Hinweises auf die Patenterteilung: **08.10.2014**
(21) Anmeldenummer: 09767945.0
(22) Anmeldetag: 20.11.2009
(51) Int. Cl.: A61Q 5/00, A61Q 5/12, A61K 8/89, A61K 8/898

(54) **KOSMETISCHE HAARSPÜLUNGEN MIT EINEM ORGANOPOLYSILOXANMODIFIZIERTEM SACCHARID UND EINEM AMINOSILIKON**
COSMETIC HAIR CONDITIONERS COMPRISING AN ORGANOPOLYSILOXANE-MODIFIED SACCHARIDE AND AN AMINO SILICONE
APRÈS-SHAMPOOINGS COSMÉTIQUES CONTENANT UN SACCHARIDE À MODIFICATION ORGANOPOLYSILOXANE ET UNE SILICONE AMINÉE

(30) Priorität: 08.12.2008 DE 102008060658
(43) Veröffentlichungstag der Anmeldung: 28.03.2012
(73) Patentinhaber: Beiersdorf AG, 20245 Hamburg (DE)
(72) Erfinder: SALADIN, Sandra, 20144 Hamburg (DE); MAHADESHWAR, Anand, 22529 Hamburg (DE); KOHUT, Michaela, 20255 Hamburg (DE)
(86) Internationale Anmeldenummer: PCT/EP2009/008282
(87) Internationale Veröffentlichungsnummer: WO 2010/075912

(56) Entgegenhaltungen:
- EP-A1- 1 550 688
- EP-B1- 1 446 440
- WO-A1-2008/046763
- WO-A1-2009/019896
- WO-A1-2009/052272
- WO-A1-2009/079610
- WO-A2-2006/127883
- JP-A- 7 041 417
- JP-A- 63 139 106

## Beschreibung

Haarspülungen werden zur Pflege nach der Reinigung der Haare angewendet. Sie sind normalerweise auf Basis von kationischen Tensiden wie beispielsweise Cetyl Trimethyl Ammonium Chlorid und Fettalkoholen wie Cetyl und/oder Stearylalkohol. Allein reicht diese Kombination jedoch nicht für eine geeignete konditionierende Pflege an Haaren wie unter anderem Geschmeidigkeit, Kämmbarkeit und Glanz aus. Aus diesem Grund werden häufiger Silikone wie beispielsweise langkettige Polydimethylsiloxane sowie auch je nach Schädigungszustand der Haare substantivere Silikone wie beispielsweise Aminosilikone eingesetzt. Es besteht weiterhin Bedarf nach Haarfaserbehandlungsmitteln, die eine avivierende Wirkung am Haar haben und auch eine gute biologische Abbaubarkeit aufweisen. Weiterhin sollen die in solchen Mitteln eingesetzten Substanzen in möglichst geringen Mengen eine ausreichende Wirksamkeit entfalten um die Umwelt zu schonen. Hierfür bieten zuckerbasierte Siloxane mehrere Vorteile an wie z. B. Wasserstoffbrückenbildungen durch die hohe Anzahl an Hydroxyl-Gruppen, Feuchtigkeitsspeichern und Geschmeidigkeit auf Oberflächen.

Ein wichtiger Faktor ist die Sensorik der Spülung während des Auftragens auf das Haar. Der Konsument verbindet mit einer reichhaltigen Haarspülung eine sehr gute Pflegewirkung. Viele Haarspülungen zeigen zwar eine gute Leistung hinsichtlich der Pflegeeigenschaften, haben jedoch eine schlechte Sensorik, vor allem im nassen Haar. Ein Nachteil der zuckerbasierten Siloxane liegt in einer leicht klebrigen Sensorik auf der nassen Haarsträhne bei der Spülungsapplikation und beim Ausspülen. Silikone, die Aminogruppen enthalten, stellen eine substantive Klasse der Silikone dar. In einer Spülungsformulierung bei pH-Werten von 4-5 sind diese Gruppen protoniert, so dass die kationische Natur des Silikons zu einer erhöhten Konditionierleistung führt. Jedoch ist die Kernseite, die Eigenschaft zur Neigung zur statischen Aufladung der Haare wenn die Silikone alleine eingesetzt werden und somit zum "Fly-Away" Effekt beim Verbraucher.

Der Stand der Technik kennt nach EP-1446440B1 über Saccharidreste funktionalisierte Organopolysiloxane und nach EP-1885331A2 den Einsatz von Saccharid-Siloxancopolymere in Personal-Care-Anwendungen. EP-1550688A1 beschreibt die Herstellung von organopolysiloxanmodifizierten Polysacchariden. EP-1097703B1 offenbart die Verwendung von Zuckersuspensionen und EP-1213316B1 kennt die kosmetische Verwendung von mit mehrwertigem Alkohol modifizierten Siloxanen. US-6066727 beschreibt die Herstellung von funktionalisierten Polysacchariden. WO-2008046763 A beschreibt die Verwendung von Siloxan-Saccharid-Copolymeren in der Kosmetik.

Es hat sich gezeigt, dass ein kosmetisches Haarbehandlungsmittel mit einem organopolysiloxanmodifiziertem Saccharid (A) und einem Aminosilikon (B) wobei (A) eine Molmasse von 22000 bis 27000 g/mol hat und die Struktur Z-S-Z aufweist wobei Z ein Monosaccharid mit 6 oder 7 Kohlenstoffatomen oder eine Glucuronsäure ist, S ein Oligodimethylsiloxan, das terminal mit den Saccharidresten über die Gruppierung (CH2)3NH(CH2)2NH über eine Amidbindung verknüpft ist, den Stand der Technik verbessert. Die Kombination von einem zuckerbasierten Silikon und von einem Aminosilikon führt zu einer erhöhten Pflegeleistung und zur besseren Kontrolle und Frisierbarkeit ohne den Nachteil der klebrigen Sensorik von den zuckerbasierten Silikonen und ohne eine statische Aufladung der Haare. Ein zuckerbasiertes Silikon mit einer für diese Erfindung geeignete Stuktur ist in CTFA als Bis-Gluconamidoethylaminopropyl Dimethicone mit dem von Dow Corning angebotenen Handelsnamen Dow Corning CE-8810 Sugar Silicone Emulsion verfügbar. Die Zubereitung kann eine Emulsion sein. Das Aminosilikon (B) ist Dow Corning 939. Das Aminosilikon kann als Emulsion oder auch als Fluid vorliegen. Es ist von Vorteil, wenn das organopolysiloxanmodifizierte Saccharid (A) in Gehalten von 0.10 bis 2.5 Gew.-%, bevorzugt 1.0 bis 2.0 Gew.-%, jeweils Aktivgehalt bezogen auf das Gesamtgewicht der Zubereitung, enthalten ist. Besonders geeignet sind Gehalte von 1,5 Gew.-% für (A) und (B). Das Verhältnis der Gehalte an (B) zu (A) ist 1:1 bis 1:3 , bevorzugt 1:1 bis 1:2.
Es ist von Vorteil, wenn der pH-Wert der Zubereitung zwischen 3,5 und 4,1 liegt. Höhere pH-Werte führen zu einer verringerten Pflegeleistung, höhere zu einer verringerten Wirksamkeit einer Konservierung.

Es ist von Vorteil, wenn Z eine Heptose oder an die Gruppierung (CH2)3NH(CH2)2NH amidisch gebunden Glucuronsäure ist. Bevorzugt ist es, wenn Z eine Heptose ist, bevorzugt Sedoheptulose, Manno-heptulose, Allo-heptulose, Talo-heptulose, Manno-heptose oder Glucoheptose. Besonders bevorzugt ist es, wenn Z eine Hexose ist, bevorzugt Glucose, Mannose, Galactose, Fructose oder Sorbose.

### Details & Ergebnisse¹:

Von einem Expertenteam (n= 3) wurden zunächst 3 haarkonditionierende Formeln sensorisch bewertet. Formeln 1 bis 3 unterscheiden sich im Silikontyp. Formel 1 enthält eine gängige Aminosilikonemulsion, Formel 2 ein zuckerbasiertes Siloxan und Formel 3 eine Mischung der Silikone. Es wurde gefunden, dass Formel 3 während des Auftragens deutlich gehaltvoller ist als Formel 1 und besser gleitend als Formel 2. Formel 3 zeigte auch die beste Nasskämmbarkeit der Reihe. Diese Ergebnisse zugunsten der Formel 3 konnten von dem Expertenteam auch im trockenen Haar bestätigt werden.
¹ Alle im Text genannten Angaben sind Aktivgehalte.

"Gehaltvoll" im Sinne dieser Schrift wird wie folgt festgelegt: Es wird gemessen, in welchem Ausmaß das Produkt als Schicht/Film zwischen der Haarsträhne und den Fingern wahrnehmbar ist. Hierbei wird eine 10er Skala verwendet bei der 10 den höchstmöglichen Gehalt darstellt.

"Gleitvermögen" im Sinne dieser Schrift bedeutet das Gleiten der Finger mit mittlerem/gleich bleibendem Druck an der Haarsträhne von oben nach unten. Hierbei wird eine 10er Skala verwendet bei der 10 das höchstmögliche Gleitvermögen darstellt.

Kämmbarkeit" im Sinne dieser Schrift ist die Messung wie leicht sich das Haar mit dem Kamm durchkämmen lässt, nachdem die Haare entwirrt wurden. Hierbei wird eine 10er Skala verwendet bei der 10 die höchstmögliche Kämmbarkeit darstellt.

**Tabelle 1**

| | inci | **1** | **2** | **3** |
|---|---|---|---|---|
| 1 | Methylparaben | 0,25 | 0,25 | 0,25 |
| 2 | Propylparaben | 0,10 | 0,10 | 0,10 |
| 3 | Aqua | Ad.100 | Ad.100 | Ad.100 |
| 4 | Cetyl Alcohol | 2,50 | 2,50 | 2,50 |
| 5 | Lactic Acid | 0,80 | 0,80 | 0,80 |
| 6 | Trisodium EDTA Solution, 20%, Beiersdorf | 1,00 | 1,00 | 1,00 |
| 7 | Sodium Chloride | 0,01 | 0,01 | 0,01 |
| 8 | Stearyl Alcohol | 4,80 | 4,80 | 4,80 |
| 9 | Amodimethicone + Trideceth-12 + Cetrimonium Chloride | 9,38 | | 4,69 |
| 10 | Oryzanol | 0,05 | 0,05 | 0,05 |
| 11 | Stearamidopropyl Dimethylamine | 2,20 | 2,20 | 2,20 |
| 12 | Gluconamidoethylaminopropylsilicone + C11-15 Pareth-40 + Butyloctanol | | 6,30 | 3,15 |

### Handelsnamen der Rohstoffe:

- 1: Nipagin M, Clariant, 99.5%
- 2: Nipasol M, Clariant, 99.5%
- 4: Nacol 16-95, Sasol, 95%
- 5: 100366 Milchsäure 90, reinst DAB, Merck, 90%
- 6: Trisodium EDTA Solution, 20%, Beiersdorf
- 8: Lanette 18, Cognis , 100%
- 9: Dow Corning 939 Cationic Emulsion, 32%
- 10: Gamma -Oryzanol, Biesterfeld, 98%
- 11: Tego Amid S18, Evonik Goldschmidt, 100%
- 12: Dow Corning CE-8810 Sugar Silicone Emulsion, 47.5%

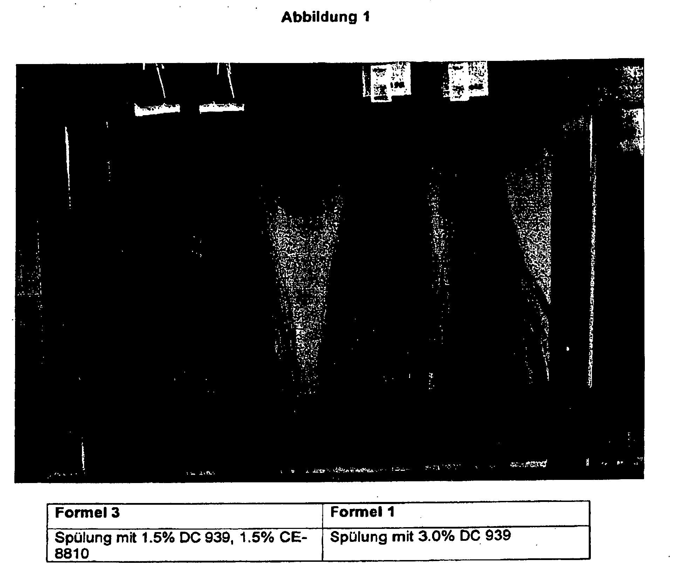

Bei der Trockenbewertung ist wie auf Abbildung 1 zu sehen, dass die linken Haarsträhnen paralleler sind als die rechten, wo es zu einer leichten statischen Aufladung kommt. Die beiden linken Haarsträhnen wurden mit Formel 3 mit der Silikonmischung behandelt.

### Beispiele für erfindungsgemäße Formulierungen:

### Spülungen

| | inci | **4** | **5** | **6** | **7** | **8** | **9** | **10** | **11** | **12** |
|---|---|---|---|---|---|---|---|---|---|---|
| 1 | Methylparaben | 0,25 | 0,25 | 0,25 | 0,25 | 0,25 | 0,25 | 0,25 | 0,25 | 0,25 |
| 2 | Propylparaben | 0,10 | 0,10 | 0,10 | 0,10 | 0,10 | 0,10 | 0,10 | 0,10 | 0,10 |
| 3 | Aqua | Ad. 100 | Ad. 100 | Ad. 100 | Ad. 100 | Ad. 100 | Ad. 100 | Ad. 100 | Ad. 100 | Ad. 10 0 |
| 4 | Citric Acid | | | | | | 0,01 | 0,01 | | |
| 5 | Cetearyl Alcohol | | | | | | 3,50 | 4,44 | | |
| 6 | Cetyl Alcohol | 2,50 | 2,50 | 2,50 | 2,50 | 2,50 | | | 2,50 | 2,00 |
| 7 | Trisodium EDTA | | | | | | | | | |
| 8 | Lactic Acid | 0,80 | 0,80 | 0,80 | 0,80 | 0,80 | | | 0,80 | 0,80 |
| 9 | Glycerin | | | | | | | 5,00 | | |
| 10 | Sodium Hydroxide | | | | | | 0,03 | 0,03 | | |
| 11 | Sodium Chloride | 0,01 | 0,01 | 0,01 | 0,01 | 0,01 | | | 0,01 | 0,01 |
| 12 | Stearyl Alcohol | 4,80 | 4,80 | 4,80 | 4,80 | 4,80 | | | 4,80 | 4,00 |
| 13 | Cetrimonium Chloride | | | | | | 2,00 | | | |
| 14 | Distearoylethyl Hydroxyethylmonium Methosulfate | | | | | | | 2,86 | | |
| 15 | Oryzanol | 0,05 | 0,05 | 0,05 | 0,05 | 0,05 | | 0,05 | 0,05 | 0,05 |
| 16 | Palmitamidoprop yltrimonium Chloride | | | | | | | 1,66 | | 0,42 |
| 17 | Coco Betaine | 0,85 | 0,85 | 0,85 | 0,85 | 0,85 | | | 0,85 | |
| 18 | Stearamidopropyl Dimethylamine | 2,00 | 2,00 | 2,00 | 2,00 | 2,00 | | | 1,50 | 2,00 |
| 19 | Amodimethicone | 3,00 | | 4,00 | | | 3,00 | 3,00 | 3,00 | 3,00 |
| 20 | Amodimethicone | | 1,00 | | | | | | | |
| 21 | Amodimethicone | | | | | 2,50 | | | | |
| 22 | Bis-Hydroxy/Methoxy Amodimethicone | | | | 1,50 | | | | | |
| 23 | Glucoamidoethylaminopropylsilico ne | 2,10 | 1,05 | 2,10 | 1,05 | 4,21 | 3,15 | 3,15 | 3,15 | 3,15 |
| 24 | Behentrimonium Methosulfate | | | | | | 2,73 | | | |

## Patentansprüche

1. Kosmetische Haarspülungen mit einem organopolysiloxanmodifiziertem Saccharid (A) und einem Aminosilikon (B) wobei (A) eine Molmasse von 22000 bis 27000 g/mol hat und die Struktur Z-S-Z aufweist, wobei
Z ein Monosaccharid mit 6 oder 7 Kohlenstoffatomen oder eine Glucuronsäure ist,
S ein Oligodimethylsiloxan, das terminal mit den Saccharidresten über die Gruppierung (CH₂)₃NH(CH₂)₂NH über eine Amidbindung verknüpft ist,
**dadurch gekennzeichnet, dass**
das Aminosilikon (B) gewählt wird unter kationischen Emulsionen Amodimethicon/Trideceth-12/Cetrimonium Chlorid (Dow Corning 939), und das Verhältnis der Gehalte an (B) zu (A) 1:1 bis 1:3 ist.

2. Zubereitung nach Anspruch 1 **dadurch gekennzeichnet, dass** das Verhältnis der Gehalte an (B) zu (A) 1:1 bis 1:2 ist.

3. Zubereitung nach Anspruch 1 oder 2 **dadurch gekennzeichnet, dass** die Zubereitung eine Emulsion ist.

4. Zubereitung nach einem der vorangehenden Patentansprüche **dadurch gekennzeichnet, dass** 0.10 bis 2.5 Gew.-%, bevorzugt 1.0 bis 2.0 Gew.-%, jeweils Aktivgehalt bezogen auf das Gesamtgewicht der Zubereitung, an organopolysiloxanmodifiziertem Saccharid (A) enthalten sind.

5. Zubereitung nach Anspruch 4 **dadurch gekennzeichnet, dass** 1.0 bis 2.0 Gew.-%, jeweils Aktivgehalt bezogen auf das Gesamtgewicht der Zubereitung, an organopolysiloxanmodifiziertem Saccharid (A) enthalten sind.

6. Zubereitung nach einem der vorangehenden Patentansprüche **dadurch gekennzeichnet, dass** der pH-Wert der Zubereitung zwischen 3,5 und 4,1 liegt.

7. Zubereitung nach einem der vorangehenden Patentansprüche **dadurch gekennzeichnet, dass** Z eine Heptose oder an die Gruppierung (CH₂)₃NH(CH₂)₂NH amidisch gebundene Glucuronsäure ist.

8. Zubereitung nach einem der vorangehenden Patentansprüche **dadurch gekennzeichnet, dass** Z eine Heptose ist, bevorzugt Sedoheptulose, Manno-heptulose, Allo-heptulose, Talo-heptulose, Manno-heptose oder Glucoheptose.

9. Zubereitung nach einem der Patentansprüche 1 bis 6 **dadurch gekennzeichnet, dass** Z eine Hexose ist, bevorzugt Glucose, Mannose, Galactose, Fructose oder Sorbose.

## Claims

1. Cosmetic hair conditioners with an organopolysiloxane-modified saccharide (A) and an arrtinosilicane (B), where (A) has a molar mass of from 22 000 to 27 000 g/mol and has the structure Z-S-Z, where
Z is a monosaccharide having 6 or 7 carbon atoms or a glucuronic acid,
S is an oligodimethylsiloxane which is terminally linked with the saccharide radicals via the group (CH₂)₃NH(CH₂)₂NH via an amide bond,
**characterized in that** the aminosilicone (B,) is selected among cationic emulsions amodimethicone/trideceth-12/ cetrimonium chloride (Dow Corning 939), and the ratio of the contents of (B) to (A) is 1:1 to 1:3.

2. Preparation according to Claim 1, **characterized in that** the ratio of the contents of (B) to (A) is 1:1 to 1:2.

3. Preparation according to Claim 1 or 2, **characterized in that** the preparation is an emulsion.

4. Preparation according to one of the preceding patent claims, **characterized in that** 0.10 to 2.5% by weight, preferably 1.0 to 2.0% by weight, in each case active content based on the total weight of the preparation, of organopolysiloxane-modified saccharide (A) are present.

5. Preparation according to Claim 4, **characterized in that** 1.0 to 2.0% by weight, in each case active content based on the total weight of the preparation, of organopolysiloxane-modified saccharide (A) are present.

6. Preparation according to one of the preceding patent claims, **characterized in that** the pH of the preparation is between 3.5 and 4.1.

7. Preparation according to one of the preceding patent claims, **characterized in that** Z is a heptose or glucuronic acid amidically bonded to the group (CH₂)₃NH(CH₂)₂NH.

8. Preparation according to one of the preceding patent claims, **characterized in that** Z is a heptose, preferably sedoheptulose, mannoheptulose, alloheptulose, taloheptulose, mannoheptose or glucoheptose.

9. Preparation according to one of patent Claims 1 to 6, **characterized in that** Z is a hexose, preferably glucose, mannose, galactose, fructose or sorbose.

## Revendications

1. Après-shampooings capillaires cosmétiques comportant un saccharide (A) modifié avec un organopolysiloxane, et un aminosilicone (B), (A) ayant une masse moléculaire de 22 000 à 27 000 g/mole et présentant la structure Z-S-Z, où
Z est un monosaccharide ayant 6 ou 7 atomes de carbone ou un acide glucuronique,
S est un oligodiméthylsiloxane qui est lié en bout de chaîne aux radicaux saccharide par une liaison amide par l'intermédiaire du groupement (CH₂)₃NH(CH₂)₂NH,
**caractérisés en ce que**
l'aminosilicone (B) est choisi parmi des émulsions cationiques amodimethicone/trideceth-12/chlorure de cétrimonium (Dow Corning 939), et le rapport des teneurs en (B) à (A) vaut de 1:1 à 1:3.

2. Préparation selon la revendication 1, **caractérisée en ce que** le rapport des teneurs en (B) à (A) vaut de 1:1 à 1:2.

3. Préparation selon la revendication 1 ou 2, **caractérisée en ce que** la préparation est un émulsion,

4. Préparation selon l'une quelconque des revendications précédentes, **caractérisée en ce que** 0,10 à 2,5 % en poids, de préférence 1,0 à 2,0% en poids, chaque fois en teneur en actif, de saccharide (A) modifié avec un organopolysiloxane, sont contenus par rapport au poids total de la préparation.

5. Préparation selon la revendication 4, **caractérisée en ce que** 1,0 à 2,0 % en poids, chaque fois en teneur en actif, de saccharide (A) modifié avec un organopolysiloxane, sont contenus par rapport au poids total de la préparation.

6. Préparation selon l'une quelconque des revendications précédentes, **caractérisée en ce que** le pH de la préparation est compris entre 3,5 et 4,1.

7. Préparation selon l'une quelconque des revendications précédentes, **caractérisée en ce que** Z est un heptose ou l'acide glucuronique lié par une liaison amide au groupement (CH₂)₃NH(CH₂)₂NH.

8. Préparation selon l'une quelconque des revendications précédentes, **caractérisée en ce que** Z est un heptose, de préférence le sédoheptulose, le manno-heptulose, l'alloheptulose, le talo-heptulose, le manno-heptose ou le glucoheptose.

9. Préparation selon l'une quelconque des revendications 1 à 6, **caractérisée en ce que** Z est un hexose, de préférence le glucose, le mannose, le galactose, le fructose ou le sorbose.
